(19) Europäisches Patentamt
European Patent Office
Office européen des brevets

(11) EP 0 563 309 B1

(12) **EUROPEAN PATENT SPECIFICATION**

(45) Date of publication and mention of the grant of the patent: **28.02.1996 Bulletin 1996/09**

(21) Application number: **92904075.6**

(22) Date of filing: **16.12.1991**

(51) Int. Cl.$^6$: **A61K 6/00**, C08F 8/44

(86) International application number: **PCT/US91/09466**

(87) International publication number: **WO 92/10987 (09.07.1992 Gazette 1992/17)**

(54) **DENTURE STABILIZING COMPOSITIONS HAVING IMPROVED TASTE**

ZUSAMMENSETZUNG MIT VERBESSERTEM GESCHMACK FÜR DIE STABILISATION VON ZAHNPROTHESEN

COMPOSITIONS SERVANT A STABILISER UNE DENTURE ARTIFICIELLE ET OFFRANT UN GOUT AMELIORE

(84) Designated Contracting States:
**AT BE CH DE DK ES FR GB GR IT LI LU NL SE**

(30) Priority: **21.12.1990 US 632291**

(43) Date of publication of application: **06.10.1993 Bulletin 1993/40**

(73) Proprietor: **RICHARDSON-VICKS, INC.**
**Shelton, CT 06484 (US)**

(72) Inventors:
- **MACKAY, Bruce, John**
  **Cincinnati, OHIO 45249 (US)**
- **SAUD, Abel**
  **Milford, CT 06460 (US)**
- **RAJAIAH, Jayanth**
  **Loveland, Ohio 45140 (US)**
- **HA, Bao, Kim**
  **Bridgeport, CT 06610 (US)**

(74) Representative: **Canonici, Jean-Jacques et al**
**B-1853 Strombeek-Bever (BE)**

(56) References cited:

| | |
|---|---|
| EP-A- 0 121 692 | EP-A- 0 396 411 |
| WO-A-91/14733 | US-A- 3 003 988 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.9.9/3.4

## Description

This invention relates to improvements in adhesives, in particular, improved denture adhesives.

Ordinary removable dentures, dental plates, and the like, comprise teeth mounted in a suitable plate or base. Dentures function as a substitute for missing teeth and serve as a replacement for all or a portion of the teeth ordinarily found in the oral cavity. Although dentures generally are skillfully prepared, often they do not fit perfectly. Moreover, no matter how satisfactory at first, after a period of time the fit of the denture becomes loose and imperfect due to natural shrinkage and changes in the gums, mucous tissues, and the like. Loose and imperfectly fitted dentures usually are corrected and stabilized by the use of a denture stabilizer. Denture stabilizers are used to fill the interstices between the dentures and the gums or tissues. Prior to placement of the denture in the oral cavity, a denture stabilizer is applied to the denture-plate surface which, for a perfect fit, should uniformly contact the gums and mucous tissues. The denture stabilizer is formulated not only for its adherent properties, but also to provide a cushion or gasket between the denture and the gums or tissues, thereby positioning the denture securely in the oral cavity.

Requirements and characteristics for a satisfactory denture stabilizing composition are many and are dictated by numerous factors. Desirably, one daily application of such a composition should function as an effective means for insulating, cushioning, and securely positioning the denture. The composition should retain its characteristics and properties in the typical powder and cream forms during storage under various climatic conditions such as high temperature and humidity; be readily and easily capable of application to the denture surface; not be irritating or uncomfortable to the user; be safe and nontoxic; have no disagreeable odor or color; have no unpalatable taste; optionally provide antiseptic and germicidal properties for preventing or inhibiting the growth of organisms ordinarily found in the mouth; and function as an agent for prevention of putrefaction or malodorous decomposition of foods or secretions lodging beneath or adjacent to the denture. The stabilizing material must be capable of imbibing water and saliva and swelling, so as to fill the interstices between the denture and the gum or mucous tissues. The stabilizer should not attack or damage the denture, as by causing a crazing of the denture-plate material. Additionally, the stabilizer should be stable to bacteria, molds and enzyme systems found in the oral cavity, and have a pH that is nonirritating to the oral mucosa, generally 5-8.5, preferably a pH around neutrality. The mechanical strength of the stabilizing mass, be it gel or colloid, formed by imbibition of water should be great enough to securely maintain the position of the denture under normal use, and not so great as to make denture removal difficult when desired, or as to damage or injure the gums, tissues or denture upon removal.

There has been a considerable effort made over many years to develop improved denture adhesives. Both synthetic and natural polymers and gums have been used singly, in combination, and in combination with various additives.

EP-A-64,672 relates to a hydrophilic denture adhesive containing an adhesive polymeric fraction comprising carboxymethylcellulose (CMC) and polyethylene oxide in a hydrophilic vehicle.

EP-A-140,486 relates to denture adhesive compositions containing a hydrophobically modified water-soluble polymer, alone or admixed with an alkali metal salt of CMC. Hydrophobically modified hydroxyalkyl celluloses and copolymers of ethylene oxide and long chain epoxyalkanes are preferred for use in the compositions.

U.S.-A-4,280,936 relates to improved denture adhesives containing a specified ratio of carboxymethylcellulose and polyethylene oxide in a mineral oil base.

U.S.-A-4,474,902 relates to improved denture adhesives containing karaya gum in a hydrophilic vehicle. See also U.S.-A-4,514,528 (equivalent to EP-A-121,692) and U.S.-A-4,518,721, relating, respectively, to improved denture adhesives containing adhesive polymeric fractions consisting of admixtures of partial salts of lower alkylvinyl ether maleic anhydride-type copolymers with carboxymethylcellulose or polyethylene oxide, as well as denture adhesives containing carboxymethylcellulose and polyethylene oxide. See also U.S.-A-4,522,956, relating to improved denture adhesives containing polyethylene oxide as the sole adhesive component in a hydrophilic vehicle comprising certain polyethylene glycols.

Other denture adhesives are described in U.S.-A-4,530,942; US-A-4,542,168; and US-A-4,569,955.

U.S.-A-4,529,748 relates to dental prosthesis adhesives formed from film-forming substances such as various cellulose derivatives, acrylate polymers, methacrylate polymers, and other film-providing substances.

U.S.-A-4,138,477 discloses oral compositions to control mouth odor containing zinc-polymer combinations formed from zinc reacted with an anionic polymer containing carboxylic, sulfonic and/or phosphonic acid radicals.

U.S.-A-3,003,988, describes certain water-sensitized, but water-insoluble, materials for stabilizing dentures which are synthetic, hydrophilic, colloidal materials comprising mixed partial salts and esters of lower alkyl (1 to 4 carbons) vinyl ether-maleic anhydride-type copolymers, said mixed partial salts and esters containing both divalent calcium and monovalent alkali (i.e., sodium, potassium and ammonium) cations.

U.S.-A-4,758,630 relates to zinc and strontium partial salts of lower alkyl ($C_1$ to $C_4$) vinyl ether-maleic acid copolymers, wherein said zinc and strontium cations are "unmixed" with any other cations or ester functions in the copolymeric salt, the remaining initial carboxyl groups being unreacted. These lower alkyl vinyl ether-maleic acid copolymers are referred to hereinafter by the abbreviated term "AVE/MA copolymer" and the methyl vinyl ether-maleic acid copolymer as "MVE/MA copolymer". Further, EP-A-396,411 discloses mixed AVE/MA copolymer salts wherein the mixed salts

comprise, as the cationic salt function, from 10% to 65% zinc or strontium cations; and from 10% to 75% calcium cations of the total initial carboxyl groups reacted.

It is known, therefore, that combinations of mixed and unmixed partial salts of lower alkyl vinyl ether-maleic anhydride-type copolymers are useful as denture adhesive compositions.

Yet, the search continues for denture stabilizers that will provide the above-described characteristics and, importantly, will maintain the secure fit of the denture over prolonged periods (10-14 hours) without the need for reapplication.

In accordance with the present invention, improved tastes characteristics are obtained in a denture stabilizing composition by using specific single mixed partial salt(s) of a lower alkyl vinyl ether-maleic acid copolymer having specific zinc, calcium, sodium, and free acid levels.

It is an object of the present invention to provide improved denture stabilizers which are easy to manufacture and that will be stable over prolonged periods in the oral cavity, yet will allow easy removal of the denture on demand.

It is a further object of the present invention to provide denture compositions which provide the user with improved sensory, such as flavor, benefits.

It is a further object to provide such stabilizers using toxicologically-acceptable, palatable materials.

It is another object herein to provide stabilizers that perform well in the presence of moisture, particularly in the presence of body fluids such as saliva, perspiration and blood.

These and other objects are secured by the present invention, in the manner disclosed hereinafter.

## SUMMARY OF THE INVENTION

The present invention encompasses stabilizer compositions comprising: the mixed partial salt of a lower alkyl vinyl ether-maleic acid copolymer consisting essentially of the repeated structural unit:

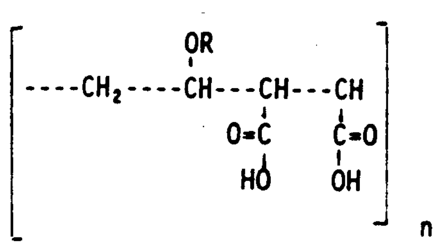

(I)

wherein R represents a $C_{1-4}$ alkyl radical, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9% zinc or strontium cations;
(b) from 0.1% to 25% sodium cations; and
(c) from 20% to 70% calcium cations
of the total initial carboxyl groups reacted, and wherein said salts have a taste index of below 0.7 and preferably below 0.6.

Also disclosed are denture stabilizing compositions comprising these mixed partial salts, as well as denture stabilizing compositions comprising a safe and adhesively effective amount of two or more denture adhesive components wherein one of said denture adhesive components is the mixed partial salt(s) of the present invention.

Preferably these mixed partial salts are used along with a water-sensitized polymeric material selected from the group consisting of natural gums, synthetic polymers, saccharide derivatives, cellulose derivatives, and mixtures thereof.

All percentages and ratios used herein relating to the neutralization of the salts of the present invention are based upon the stoichiometric percent of the cations present in the salt. All other percentages and ratios used herein are by weight, unless otherwise specified.

DETAILED DESCRIPTION OF THE INVENTION

The polymeric salts of the present invention are the mixed partial salt of a lower alkyl vinyl ether-maleic acid copolymer consisting essentially of the repeated structural unit:

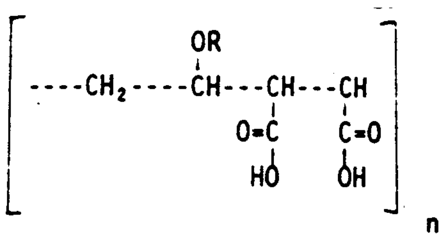

(I)

wherein R represents a $C_{1-4}$ alkyl radical, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9% zinc or strontium cations;
(b) from 0.1% to 25% sodium cations; and
(c) from 20% to 70% calcium cations
of the total initial carboxyl groups reacted and wherein said salts have a taste index of below 0.7 and preferably below 0.6.

R is preferably methyl.

Preferably, these mixed partial salts comprise from 0.1% to 5% zinc or strontium cations (preferably zinc) and from 5% to 10% sodium cations, and 10% to 30% free acid.

The taste index of the salts of the present invention is determined from the following calculation based upon the stoichiometric percent of the cations present in the salt:

$$\text{Taste Index} = 1 - \frac{A+B+C}{3}$$

where A,B,C range from 0 to 1 and are defined by:

$$A = \frac{\%\ Na}{10}$$

$$B = \frac{\%\ Zn - 17.5}{-17.5}$$

$$C = \frac{\%\ \text{Free Acid} - 35}{-15}$$

For example, a composition containing 10% sodium, 5% zinc and 55% calcium with 30% free acid would yield a taste index of 0.32 as follows:

$$A = \frac{10}{10} = 1$$

$$B = \frac{5 - 17.5}{-17.5} = 0.71$$

$$C = \frac{30 - 35}{-15} = 0.33$$

$$\text{Taste Index} = 1 - \frac{A+B+C}{3} = 1 - \frac{1 + 0.71 + 0.33}{3} = 0.32$$

The subject polymeric salts are advantageously prepared by the interaction of the AVE/MA copolymer (I) with cationic calcium, sodium and either zinc or strontium compounds having a functional group typical of reactants of carboxylic acid, such as, for example, the hydroxide, acetate, carbonate, halide, lactate, etc. in an aqueous medium. In a preferred embodiment, the oxide of zinc and the hydroxide of calcium and sodium are utilized. Since zinc hydroxide is not commercially available, its use as a reactant is readily and more economically accomplished by employing an aqueous slurry of particulate zinc oxide which, although practically insoluble in water, provides hydration to zinc hydroxide on the particulate surface. Calcium and sodium hydroxides as well as strontium hydroxide, on the other hand, are available in either crystalline or powder form and are soluble in about 50 parts water. Aqueous solutions of strontium oxide, however, which form the hydroxide when treated with water (caution: heat evolution), may also be used. Strontium Carbonate powder may also be used.

Anions that form toxic, irritating or contaminating by-products should be avoided, or special precautions and treatment provided to assure the removal and absence of such by-products from the polymeric salt end-product. The particular compound used should be substantially pure to assure obtaining a substantially pure, substantially off-white polymeric salt end-product.

The lower alkyl vinyl ether maleic acid (AVE/MA) copolymers (I) are readily obtained by copolymerizing a lower alkyl vinyl ether monomer, such as methyl vinyl ether, ethyl vinyl ether, divinyl ether, propyl vinyl either and isobutyl vinyl ether, with maleic anhydride to yield the corresponding lower alkyl vinyl ether-maleic anhydride copolymer which is readily hydrolyzable to the acid copolymer (I). Both anhydride and acid forms are also available from commercial suppliers. For example, the GAF Corporation, Wayne, New Jersey, provides both the polymeric free acid form (I) and the corresponding anhydride form under its "GANTREZ" trademark as the "GANTREZ S Series" and "GANTREZ AN Series", respectively. In the former acid series, the GANTREZ S-97 (M.W.=50,000) is particularly suitable, and, in the latter anhydride series, the GANTREZ AN-149 (M.W.=50,000), the GANTREZ AN-169 (M.W.=67,000) and the GANTREZ AN-179 (M.W.=80,000) copolymers are particularly suitable. Said acid and anhydride forms of AVE/MA copolymers, having an average molecular weight of from about 50,000 to about 80,000 (as measured by membrane osmometry in 2-butanone 1-10 grams/1000 ml solution), are also characterized by having the previously described specific viscosity parameter of more than 1.2. When the anhydride copolymer dissolves in water, the anhydride linkage is cleaved so that the highly polar, polymeric free acid (I) is formed. Accordingly, the anhydride form, which is relatively less expensive than the acid form, may be used as a convenient and cheaper precursor for the acid. Elevated temperatures may be advantageously employed to enhance the rate of anhydride-to-acid hydrolysis.

In general, the lower alkyl vinyl ether-maleic acid copolymer (I), or its corresponding anhydride, is added to water preheated to about 70-80°C with vigorous stirring to form a homogeneous mixture. If the anhydride precursor is utilized, it is recommended that the aqueous mixture be further heated to about 90°C with stirring to ensure complete hydrolysis of the anhydride to the acid form. Heating is then discontinued although mixing is continued until the batch turns clear with a simultaneous decrease in viscosity (about 65-75°C). An aqueous solution of the cationic zinc or strontium salt forming compound, or, for example, an aqueous dispersion of particulate zinc oxide is combined with calcium hydroxide in the form of a slurry, in an amount sufficient to provide the desired cationic zinc and calcium content desired in the end-product, is separately prepared at ambient temperature and slowly added to the hot polymeric acid solution with continuous vigorous mixing so as to prevent localized precipitation of the cationic polymeric salt. After the calcium and zinc have reacted an aqueous solution of sodium hydroxide is added slowly, in the amount sufficient to provide the cation sodium content desired in the end product. After addition is complete, mixing is continued to ensure that all the salt forming compounds are reacted with the copolymer.

Alternatively, an aqueous solution containing the zinc, sodium, and calcium source is preheated to 70-80°C with vigorous stirring to form a homogeneous slurry. The lower alkyl vinyl ether-maleic acid copolymer (I) or its corresponding anhydride is then added to the slurry while further heating to 90°C and stirring to ensure complete hydrolysis. Alternatively, the AVE/MA copolymer, calcium, and strontium or zinc oxide powders are slurried in water at 25°C and subsequently heated to 80°C - 90°C for reaction to occur. Upon completion of this reaction step an aqueous solution of sodium hydroxide is slowly added.

The zinc (or strontium), sodium and calcium cations in the resultant mixed partial salt of AVE/MA copolymers should be sufficient to give a neutralization ranging from 20% to 70%, preferably from 40% to 70% calcium cations, and from 0.1% to 9.9%, preferably from 0.1% to 5% zinc or strontium cations, and from 0.1% to 25%, preferably from 5% to 10% sodium cations resulting in a salt containing free acid in the range of from 10% to 40%, preferably from 10% to 30%.

The reaction batch is then dried such as by shallow drying trays in a convection oven maintained at about 70°C with hot air circulation to evaporate the water content and recover the polymeric salt product in dry form. Alternatively, the reaction batch is then transferred to drum dryers maintained at 80-100 PSIG with hot steam to evaporate the water content and recover the polymeric salt in the flake form.

The resulting flakes may be subjected to milling and screening to yield the desired physical properties to provide satisfactory denture stabilizing properties.

Said salts are friable so that appropriate particle size and bulk density can be obtained. For best results, drum dried flakes should be milled to a preferred bulk density of about 0.5 to about 1.2 more preferably about 0.6 to about 1.1 and most preferably about 0.7 to about 1.0 grams per cubic centimeter while maintaining a specific surface area of about 0.5 to about 2.5, more preferably about 0.6 to about 2.0, and most preferably about 0.7 to about 1.5 square meters per

gram. Ground particles should be capable of passage through a 74-105 µm sieve (140- to 200-mesh, U.S.B.S. series) and preferably are less than 0.3 millimeters in their largest dimension. Bulk densities are measured according to ASTM method 8-52 (02.05).

The subject zinc or strontium, sodium and calcium AVE/MA copolymer salts have exceptional taste and adhesive qualities when contacted with water or saliva such that they are extremely useful as denture adhesive materials with improved taste in denture stabilizing compositions. For such use the salt in particulate form is preferably characterized by a particle size of at least minus 105 µm (140-mesh U.S.B.S.) sieve; a bulk density greater than 0.3 gram per cubic centimeter and preferably higher then 0.6 grams per cubic centimeter; and a pH between 3 and 8, and preferably between 5 and 7.5 the pH being determined on a one percent by weight dispersion in water.

Each of the subject calcium/sodium/zinc or strontium AVE/MA copolymer salts may be utilized in effective adhesive amounts, preferably at least 25 percent by weight, as the sole adhesive component or as a co-adhesive in joint usage with other active adhesive components in denture stabilizing compositions.

It is preferred that said calcium/sodium/zinc or strontium copolymer salt be used along with a co-adhesive in denture stabilizing compositions. Preferably, the co-adhesive is a polymeric material selected from natural gums, synthetic polymers, saccharide derivatives, cellulose derivatives, and mixtures thereof. In general, from about 15 to about 70 percent, based on the total weight of the composition, of said mixed calcium/sodium/zinc or strontium salt is present.

Preferred co-adhesives include a water-soluble hydrophilic colloid or polymer having the particular property of swelling upon exposure to moisture to form a mucilaginous mass. Such adhesive materials include both natural gums and synthetic polymeric gums and, among those commonly employed in denture stabilizing compositions and which are also suitable herein co-adhesive action with the subject mixed AVE/MA copolymer salts, there may be mentioned, for example, karaya gum, gelatin, algin, sodium alginate, tragacanth, methylcellulose, acrylamide polymers, ethylene oxide polymers, polyvinylpyrrolidone, cationic polyarylamide polymers and, as the most preferred, sodium carboxymethylcellulose and mixed partial salts of poly(vinyl methylether-maleic acid) copolymer.

Accordingly, a preferred aspect of the subject invention provides a denture stabilizing composition having as a stabilizing component an effective adhesive amount of a mixed partial salt of a lower alkyl vinyl ether-maleic acid copolymer consisting essentially of the repeated structural unit:

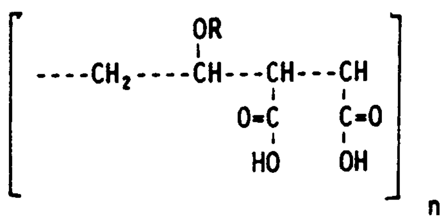

(I)

wherein R represents a $C_1$ to $C_4$ alkyl radical, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9% zinc or strontium cations;
(b) from 0.1% to 25% sodium cations; and
(c) from 20% to 70% calcium cations
of the total initial carboxyl groups reacted and wherein said salts have a taste index of below 0.7 and preferably below 0.6.

Another preferred aspect of this invention provides a denture stabilizing composition comprising a safe and adhesively effective amount of at least two denture adhesive components, wherein one of said denture adhesive components is the mixed partial salt of a lower alkyl vinyl ether-maleic acid copolymer described above. Preferably the co-adhesive is as described above.

The compositions of the present invention can optionally include from 0.01% to 5% of one or more components which provide the user with sensory, including flavor, benefits. Suitable components include menthol, menthyl lactate, spearmint oil, peppermint oil, leaf alcohol, as well as those paramenthane carboxyamide flavoring agents available from Wilkinson-Sword (such as WS-3) which are described in U.S.-A-4,136,163.

The compositions of the present invention are manufactured in an art-recognized manner known to those skilled in the art, such as in a powder, cream, ointment, liquid, paste, water or film. The compositions of the present invention are preferably manufactured using appropriate micronization such as fluid energy or air jet or hammer milling of drum dried

mixed partial salts of AVE/MA copolymer. Suitable examples of such formulations are disclosed in U.S.-A-4,518,721 and U.S.-A-4,514,528.

It is to be recognized that the adhesive salts of the present invention can be used for a wide variety of general adhesive uses including, but not limited to, pharmaceutical uses (e.g., oral drug delivery and topical bandages); and aqueous adhesives (e.g., where adhesiveness in the presence of water is required).

The following examples illustrate embodiments of the subject invention wherein both essential and optional ingredients are combined.

Example I

Into a reaction vessel equipped with a high speed stirrer and containing 85.1 parts (7.7 kg) of purified water heated to 85°C, is slowly added 0.1 parts (9 grams) of zinc oxide and calcium hydroxide 1.1 parts (98.9 grams). After addition is complete, the temperature of the slurry is kept constant with high speed mixing. While keeping heat and mixing constant add 3.9 parts (347.2 grams) of methyl vinyl ether-maleic anhydride copolymer to the reaction vessel containing the alkali dispersion over a 15 minute period. At about 15 minutes the resulting adhesive polymeric dispersion is characterized by an increase in viscosity, and a decrease and stabilization of the reaction pH of the dispersion of said material in water, said material consisting of mixed partial calcium zinc salt of methyl vinyl ethermaleic acid copolymer. Temperature and mixing remain constant for 60 minutes.Next, 890 grams of a 1% solution of sodium hydroxide is slowly added over a 30 minute period and the reaction is allowed to go to completion as indicated by the stabilization of the reaction pH.

The resultant solution of the calcium zinc sodium salt of methyl vinyl ether-maleic acid (MVE/MA) copolymer is then transferred to shallow stainless steel drying trays and the trays placed in a hot air convection oven at 70 °C for a sufficient time to evaporate the water content (about 16-18 hours). The thus obtained dried calcium zinc sodium MVE/MA copolymer salt is then ground in a milling apparatus and screened through a 74 µm (140-mesh) sieve and then through a 104 µm (200 mesh) sieve (U.S.B.S. sieve series). The powder would have a bulk density of about 0.6-1.2 gram per cubic centimeter. Analysis of the salt would indicate about 60 percent of the total carboxyl groups would be neutralized with calcium, 5 percent neutralized with zinc and 5% neutralized with sodium with 30% of the carboxyl groups remaining unreacted. This particular salt will be referred to hereinafter by the abbreviated term, "60% Ca/5% Zn/5% Na partial salt of MVE/MA copolymer". This salt has a taste index of 0.49.

The product, when used in conjunction with conventional denture adhesives and applied to wet dentures with normal usage, provides denture stabilizing and taste characteristics superior to those obtained by the particular conventional denture adhesive itself.

Example II

The procedure of Example I is repeated except that the following amounts of reactants are employed: 3.9 parts (347 grams) of the anhydride copolymer, 75.4 parts (6.8 kg) purified water; and 0.1 parts (9 grams) of zinc oxide; 1.8 Kg of a 1% solution of sodium hydroxide and 1.00 parts (90.5 g) calcium hydroxide.

The resultant powder would have an bulk density of about 0.6-1.2 grams per cubic centimeter. Analysis of the salt would indicate about 55 percent calcium neutralization of the total initial carboxyl groups in the copolymer salt molecule; 5 percent neutralization with zinc and 10% neutralization with sodium and will be referred to hereinafter by the abbreviated term "55% calcium/5% zinc/10% sodium partial salt of MVE/MA copolymer". This salt would have a taste index of 0.32.

Example III

By following the general procedure of Example I, except that an appropriate amount of zinc oxide is utilized to provide the tabulated zinc substitution, the following calcium/zinc/sodium salts of MVE/MA copolymer are obtained:

| Sodium | Calcium | Zinc | Taste Index |
|---|---|---|---|
| 20 | 40 | 9.9 | .08 |
| 10 | 60.5 | 2.5 | .20 |
| 10 | 65 | 2.5 | .10 |

Each of the indicated MVE/MA copolymer salts, which would have a bulk density for the minus 74 µm (140-mesh) U.S.B.S. sieve powder greater than 0.5 gram per cubic centimeter, will provide markedly beneficial denture stabilizing and taste characteristics. Each of the indicated salts may be abbreviated by the percent of calcium/percent of zinc/percent sodium neutralization as done in Examples I and II.

Example IV

Denture stabilizing powder compositions are prepared by blending together the following:

| | | % w/w |
|---|---|---|
| A. | Karaya gum | 53 |
| | Sodium carboxymethylcellulose | 16 |
| | Sodium borate | 7 |
| | 60% Ca/5% Zn/5% Na/30% Free Acid partial salt of PVM/MA copolymer (taste index = 0.49) | 24 |
| | | 100 |
| B. | Sodium alginate | 55 |
| | Sodium carboxymethylcellulose | 10 |
| | Polyvinylpyrrolidone (average M.W.=90,000) | 15 |
| | 55% Ca/5% Zn/10% Na/30% Free Acid partial salt of PVM/MA copolymer (taste index = 0.32) | 20 |
| | | 100 |

In use, the above powders (typically 0.1-1.0 g) are placed on a premoistened denture, allowed to hydrate briefly, and the denture is inserted in the mouth and pressed into place, all in the manner of denture adhesives well-known in the art.

Example V

Liquid-type denture stabilizing compositions are prepared by mixing together the following:

| | % w/w | |
|---|---|---|
| | A | B |
| Mineral oil, heavy | 44.9 | 43.9 |
| Petrolatum | 3.0 | 5.0 |
| Colloidal silica | 1.5 | 1.0 |
| Sodium carboxymethylcellulose | 35.0 | 20.0 |
| Menthol | 0.1 | 0.1 |
| 60% Ca/5% Zn/5% Na/30% Free Acid partial salt of MVE/MA copolymer (taste index = 0.49) | 15.5 | 30.0 |
| | 100.0 | 100.0 |

In use, the above liquid compositions (typically 0.1-1 g) are placed on a premoistened denture, and the denture is inserted in the mouth and pressed into place, all in the manner of denture adhesives well-known in the art.

Example VI

Cream-type denture stabilizing compositions are prepared by mixing together the following:

| | % w/w | |
|---|---|---|
| | A | B |
| Mineral oil, heavy | 24.824 | 24.824 |
| Sodium carboxymethylcellulose | 22.000 | 22.000 |
| Petrolatum | 19.016 | 19.016 |
| Silicon dioxide, colloidal | 1.100 | 1.100 |
| Colorant (oil soluble red color dispersion) | 0.060 | 0.600 |
| 60%Ca/5%Zn/5% Na partial mixed salt of PVM/MA copolymer (taste index = 0.49) | 33.000 | ----- |
| 60.5%Ca/2.5%Zn/10% Na partial mixed salt of PVM/MA copolymer (taste index = 0.2) | ------ | 33.000 |

In use, the above compositions (typically 0.1-2 g) are placed on a premoistened denture, and the denture is inserted in the mouth and pressed into place, all in the manner of denture adhesives well-known in the art.

**Claims**

1. The mixed partial salt of a lower alkyl vinyl ether-maleic acid copolymer consisting essentially of the repeated structural unit:

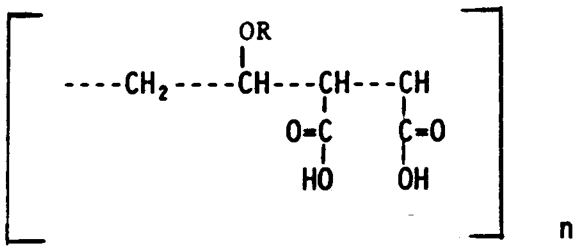

(I)

wherein R represents a $C_1$ to $C_4$ alkyl radical, preferably methyl, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9%, preferably from 0.1% to 5% zinc or strontium cations, preferably zinc cations;
(b) from 0.1% to 25%, preferably from 5% to 10% sodium cations; and
(c) from 20% to 70% calcium cations, preferably from 40% to 70%
of the total initial carboxyl groups reacted and wherein said salt has a taste index of below 0.7, preferably below 0.6.

2. A denture stabilizing composition having as a stabilizing component at least 25% by weight of a mixed partial salt of a lower alkyl vinyl ether-maleic acid copolymer consisting essentially of the repeated structural unit:

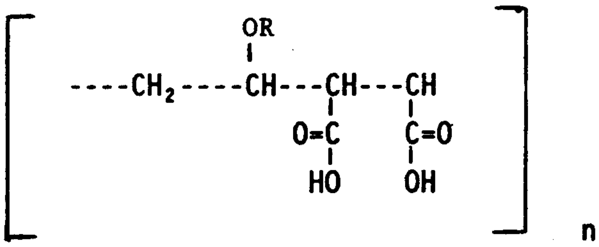

(I)

wherein R represents a $C_1$ to $C_4$ alkyl radical, preferably methyl, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9%, preferably from 0.1% to 5% zinc or strontium cations, preferably zinc cations;
(b) from 0.1% to 25%, preferably from 5% to 10% sodium cations; and
(c) from 20% to 70% calcium cations, preferably from 40% to 70%
of the total initial carboxyl groups reacted and wherein said salt has a taste index of below 0.7, preferably below 0.6.

3. A denture stabilizing composition with improved taste comprising at least two denture adhesive components, wherein one of said denture adhesive components is the mixed partial salt of a lower alkyl vinyl ether-maleic acid copolymer consisting essentially of the repeated structural unit:

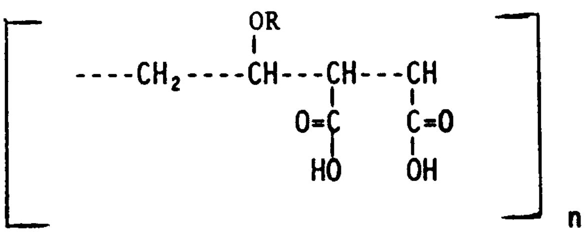

(I)

wherein R represents a $C_1$ to $C_4$ alkyl radical, preferably methyl, n is an integer greater than one representing the number of repeated occurrences of said structural unit in a molecule of said copolymer and n is large enough to characterize said copolymer as having a specific viscosity larger than 1.2, the specific viscosity being determined in methyl ethyl ketone at 25°C, said partial salts containing from 10% to 40% free acid, and as the cationic salt function:

(a) from 0.1% to 9.9%, preferably from 0.1% to 5% zinc or strontium cations, preferably zinc cations;
(b) from 0.1% to 25%, preferably from 5% to 10% sodium cations; and
(c) from 20% to 70% calcium cations, preferably from 40% to 70%
of the total initial carboxyl groups reacted and wherein said salt has a taste index of below 0.7, preferably below 0.6; the mixed partial salt being present in an amount of at least 25% by weight of the composition.

4. The denture stabilizing composition of Claim 2 or Claim 3 wherein the composition further comprises a co-adhesive is selected from natural gums, synthetic polymers, saccharide derivatives, cellulose derivatives, and mixtures thereof.

5. The denture stabilizing composition of any of the preceding Claims which further comprises from .01% to 5.0% of menthol, menthyl lactate, peppermint oil, spearmint oil, leaf alcohol, and paramenthane carboxyamides, and mixtures thereof.

**Patentansprüche**

1. Gemischtes Teilsalz von einem Niederalkylvinylether-Maleinsäure-Copolymer, welches im wesentlichen aus der sich wiederholenden Struktureinheit:

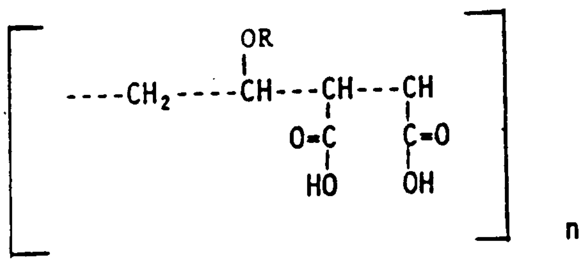

(I)

besteht, worin R einen $C_1$-$C_4$-Alkylrest, vorzugsweise Methyl darstellt, n eine ganze Zahl größer 1 ist, welche die Anzahl des wiederholten Auftretens der genannten Struktureinheit in einem Molekül des genannten Copolymers bedeutet, und worin n groß genug ist, um das genannte Copolymer als solches zu charakterisieren, welches ein relatives Viskositätsinkrement größer 1,2 aufweist, wobei das relative Viskositätsinkrement in Methylethylketon bei 25°C ermittelt wird, welche Teilsalze 10% bis 40% freie Säure und als kationische Salzfunktion:

   (a) 0,1% bis 9,9%, vorzugsweise 0,1% bis 5% Zink- oder Strontiumkationen, vorzugsweise Zinkkationen;
   (b) 0,1% bis 25%, vorzugsweise 5% bis 100% Natriumkationen; und
   (c) 20% bis 70% Calciumkationen, vorzugsweise 40% bis 70%, enthalten, bezogen auf die gesamten umgesetzten ursprünglichen Carboxylgruppen,

und wobei das genannte Salz einen Geschmacksindex unter 0,7, vorzugsweise unter 0,6 besitzt.

2. Stabilisierende Zusammensetzung für Zahnprothesen, welche als stabilisierende Komponente mindestens 25 Gew.-% eines gemischten Teilsalzes von einem Niederalkylvinylether-Maleinsäure-Copolymer enthält, welches im wesentlichen aus der sich wiederholenden Struktureinheit:

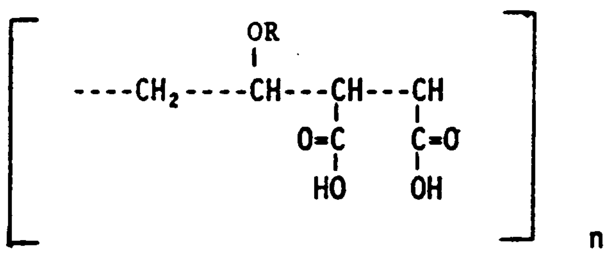

(I)

besteht, worin R einen $C_1$-$C_4$-Alkylrest, vorzugsweise Methyl darstellt, n eine ganze Zahl größer 1 ist, welche die Anzahl des wiederholten Auftretens der genannten Struktureinheit in einem Molekül des genannten Copolymers bedeutet, und worin n groß genug ist, um das genannte Copolymer als solches zu charakterisieren, welches ein relatives Viskositätsinkrement größer 1,2 aufweist, wobei das relative Viskositätsinkrement in Methylethylketon bei 25°C ermittelt wird, welche Teilsalze 10% bis 40% freie Säure und als kationische Salzfunktion:

   (a) 0,1% bis 9,9%, vorzugsweise 0,1% bis 5% Zink- oder Strontiumkationen, vorzugsweise Zinkkationen;
   (b) 0,1% bis 25%, vorzugsweise 5% bis 10% Natriumkationen; und
   (c) 20% bis 70% Calciumkationen, vorzugsweise 40% bis 70%, enthalten, bezogen auf die gesamten umgesetzten ursprünglichen Carboxylgruppen,

und wobei das genannte Salz einen Geschmacksindex unter 0,7, vorzugsweise unter 0,6 besitzt.

3. Stabilisierende Zusammensetzung für Zahnprothesen mit einem verbesserten Geschmack, umfassend mindestens zwei Haftmittelkomponenten für Zahnprothesen, worin eine der genannten Haftmittelkomponenten für Zahnprothesen das gemischte Teilsalz von einem Niederalkylvinylether-Maleinsäure-Copolymer ist, welches im wesentlichen

aus der sich wiederholenden Struktureinheit:

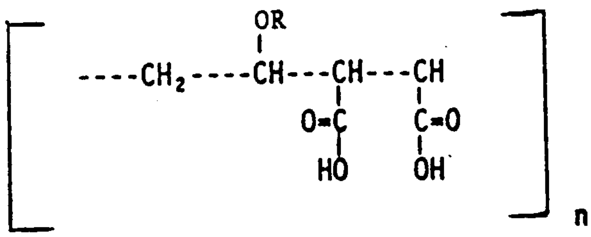

(I)

besteht, worin R einen $C_1$-$C_4$-Alkylrest, vorzugsweise Methyl darstellt, n eine ganze Zahl größer 1 ist, welche die Anzahl des wiederholten Auftretens der genannten Struktureinheit in einem Molekül des genannten Copolymers bedeutet, und worin n groß genug ist, um das genannte Copolymer als solches zu charakterisieren, welches ein relatives Viskositätsinkrement größer 1,2 aufweist, wobei das relative Viskositätsinkrement in Methylethylketon bei 25°C ermittelt wird, welche Teilsalze 10% bis 40% freie Säure und als kationische Salzfunktion:

(a) 0,1% bis 9,9%, vorzugsweise 0,1% bis 5% Zink- oder Strontiumkationen, vorzugsweise Zinkkationen;
(b) 0,1% bis 25%, vorzugsweise 5% bis 10% Natriumkationen; und
(c) 20% bis 70% Calciumkationen, vorzugsweise 40% bis 70%, enthalten, bezogen auf die gesamten umgesetzten ursprünglichen Carboxylgruppen,

und wobei das genannte Salz einen Geschmacksindex unter 0,7, vorzugsweise unter 0,6 besitzt; wobei das gemischte Teilsalz in einer Menge von mindestens 25 Gew.-% der Zusammensetzung vorliegt.

**4.** Stabilisierende Zusammensetzung für Zahnprothesen nach Anspruch 2 oder Anspruch 3, wobei die Zusammensetzung ferner ein Co-Haftmittel enthält, welches unter natürlichen Gummen, synthetischen Polymeren, Saccharidderivaten, Cellulosederivaten und Gemischen hievon ausgewählt ist.

**5.** Stabilisierende Zusammensetzung für Zahnprothesen nach einem der vorstehenden Ansprüche, welche ferner 0,01% bis 5,0% an Menthol, Menthyllactat, Pfefferminzöl, Spearminzenöl, Blattalkohol und Paramenthancarboxyamiden und Gemischen hievon umfaßt.

## Revendications

**1.** Sel partiel mixte d'un copolymère éther d'alkyle (inférieur) et de vinyle/acide maléique, essentiellement constitué du motif récurrent structural:

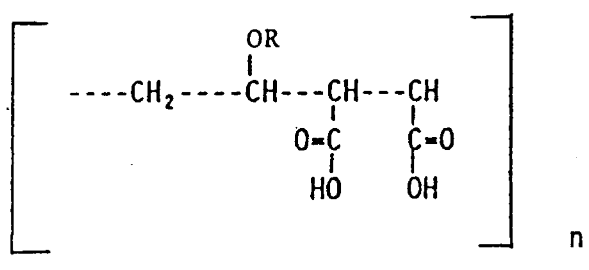

(I)

dans lequel R représente un radical alkyle en $C_1$ à $C_4$, de préférence méthyle, n est un nombre entier supérieur à celui représentant le nombre de répétitions dudit motif structural dans une molécule dudit copolymère, et n est suffisamment grand pour que ledit copolymère soit caractérisé par une viscosité spécifique supérieure à 1,2, la viscosité spécifique étant déterminée dans la méthyléthylcétone à 25°C, lesdits sels partiels contenant de 10% à 40% d'acide libre et, comme fonction sel cationique:

(a) de 0,1% à 9,9%, de préférence de 0,1% à 5%, de cations zinc ou strontium, de préférence de cations zinc;
(b) de 0,1% à 25%, de préférence de 5% à 10%, de cations sodium; et
(c) de 20% à 70% de cations calcium, de préférence de 40% à 70% des groupes carboxyle totaux initiaux ayant réagi,
et ledit sel ayant un indice de goût inférieur à 0,7, de préférence inférieur à 0,6.

2. Composition de stabilisation d'une prothèse dentaire possédant, comme constituant stabilisant, au moins 25% en poids d'un sel partiel mixte d'un copolymère éther d'alkyle (inférieur) et de vinyle/acide maléique, essentiellement constitué du motif récurrent structural:

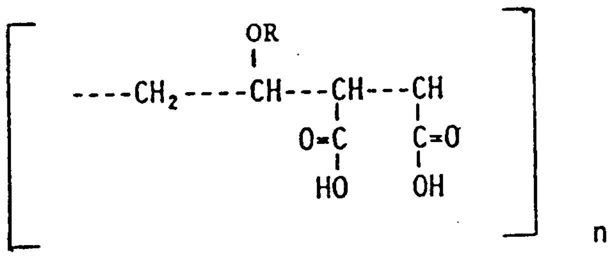

(I)

dans lequel R représente un radical alkyle en $C_1$ à $C_4$, de préférence méthyle, n est un nombre entier supérieur à celui représentant le nombre de répétitions dudit motif structural dans une molécule dudit copolymère, et n est suffisamment grand pour que ledit copolymère soit caractérisé par une viscosité spécifique supérieure à 1,2, la viscosité spécifique étant déterminée dans la méthyléthylcétone à 25°C, lesdits sels partiels contenant de 10% à 40% d'acide libre et, comme fonction sel cationique:

(a) de 0,1% à 9,9%, de préférence de 0,1% à 5%, de cations zinc ou strontium, de préférence de cations zinc;
(b) de 0,1% à 25%, de préférence de 5% à 10%, de cations sodium; et
(c) de 20% à 70% de cations calcium, de préférence de 40% à 70% des groupes carboxyle totaux initiaux ayant réagi,
et ledit sel ayant un indice de goût inférieur à 0,7, de préférence inférieur à 0,6.

3. Composition de stabilisation d'une prothèse dentaire ayant un goût amélioré, comprenant au moins deux constituants adhésifs de prothèse dentaire, dans laquelle l'un desdits constituants adhésifs de prothèse dentaire est le sel partiel mixte d'un copolymère éther d'alkyle (inférieur) et de vinyle/acide maléique, essentiellement constitué du motif récurrent structural:

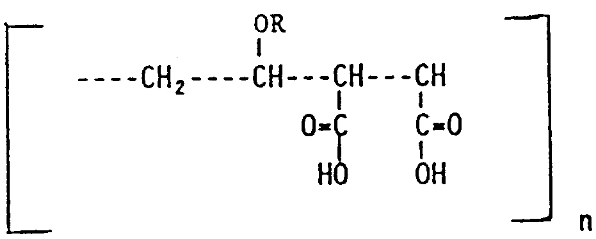

(I)

dans lequel R représente un radical alkyle en $C_1$ à $C_4$, de préférence méthyle, n est un nombre entier supérieur à celui représentant le nombre de répétitions dudit motif structural dans une molécule dudit copolymère, et n est suffisamment grand pour que ledit copolymère soit caractérisé par une viscosité spécifique supérieure à 1,2, la viscosité spécifique étant déterminée dans la méthyléthylcétone à 25°C, lesdits sels partiels contenant de 10% à 40% d'acide libre et, comme fonction sel cationique:

(a) de 0,1% à 9,9%, de préférence de 0,1% à 5%, de cations zinc ou strontium, de préférence de cations zinc;
(b) de 0,1% à 25%, de préférence de 5% à 10%, de cations sodium; et
(c) de 20% à 70% de cations calcium, de préférence de 40% à 70% des groupes carboxyle totaux initiaux ayant réagi,
et ledit sel ayant un indice de goût inférieur à 0,7, de préférence inférieur à 0,6; le sel partiel mixte étant présent à raison d'au moins 25% en poids de la composition.

4. Composition de stabilisation d'une prothèse dentaire selon la revendication 2 ou la revendication 3, dans laquelle la composition comprend, en outre, un coadhésif choisi parmi les gommes naturelles, les polymères synthétiques, les dérivés de saccharide, les dérivés de cellulose, et leurs mélanges.

5. Composition de stabilisation d'une prothèse dentaire selon l'une quelconque des revendications précédentes, qui comprend, en outre, de 0,01% à 5,0% de menthol, de lactate de menthyle, d'essence de menthe poivrée, d'essence de menthe verte, d'alcool de feuille et de carboxylamides de paramenthane, et leurs mélanges.